# EUROPÄISCHE PATENTSCHRIFT

(11) **EP 0 876 822 B1**
(45) Veröffentlichungstag und Bekanntmachung des Hinweises auf die Patenterteilung: **24.07.2002**
(21) Anmeldenummer: 98103978.7
(22) Anmeldetag: 06.03.1998
(51) Int. Cl.: A61M 1/36, B04C 3/00, B04C 3/06

(54) **Vorrichtung zum Ausscheiden von Gas aus gashaltigem Blut**
Apparatus for separating gas from blood
Appareil pour séparer un gaz du sang

(30) Priorität: 09.05.1997 DE 19719555
(43) Veröffentlichungstag der Anmeldung: 11.11.1998
(73) Patentinhaber: Convergenza AG, 9490 Vaduz (LI)
(72) Erfinder: Brockhoff, Alexander, 9494 Schaan (LI); Plechinger, Hans, Cranbrook, B.C.VIC 6J5 (CA)
(74) Vertreter: Vetter, Ewald Otto, Dipl.-Ing.

(56) Entgegenhaltungen:
- EP-A- 0 728 509
- EP-A- 0 778 031
- FR-A- 2 274 363
- GB-A- 1 526 509

## Beschreibung

Die Erfindung betrifft eine Vorrichtung zum Ausscheiden von Gas aus gashaltigem Blut gemäß dem Oberbegriff von Patentanspruch 1.

Eine Vorrichtung dieser Art ist aus der GB-A-2 063 108 bekannt. Weitere Vorrichtungen zum Trennen von Gas aus gashaltigem Blut sind aus den US-A-3 785 380, 4 368 118, 4 388 922 und 5 451 321 sowie den DE-C-36 24 363 und 36 41 644 und DE-A-43 29 385 bekannt.

Wenn einem Patienten Blut zugeführt wird, dann sollte das Blut keine Luft und keine anderen Gase enthalten, auch nicht in Form von mikro-kleinen Gasbläschen. Die Zufuhr des Blutes zu einem Patienten erfolgt durch eine Druckpumpe. Dies ist das bevorzugte Anwendungsgebiet der Erfindung. Dies schließt nicht aus, daß die Erfindung auch angewendet wird, um Luft von Blut zu trennen, welches an einer Wunde von einem Patienten abgesaugt wird, weil hier häufig nicht vermieden werden kann, daß an der Wunde auch Luft in den Blutstrom mit eingesaugt wird. Die Luft sollte möglichst schnell und nahe bei der Wunde wieder vom dem Blut getrennt werden, weil es sonst zu einer Schädigung des Blutes führen kann. Andere Anwendungsgebiete der Erfindung sind die Trennung von Gas aus gashaltigem Blut, welches von einem Gerät zu einem anderen Gerät oder Behälter transportiert wird.

Mit der Erfindung soll die Aufgabe gelöst werden, den Wirkungsgrad der Gastrennung zu verbessern und insbesondere eine Vorrichtung zu schaffen, mit welchen auch mikro-kleine Gasbläschen aus gashaltigem Blut ausgeschieden werden können, auch dann, wenn das gashaltige Blut in großer Menge pro Zeiteinheit gefördert wird.

Diese Aufgabe wird durch die kennzeichnenden Merkmale von Anspruch 1 gelöst.

Weitere Merkmale der Erfindung sind in den Unteransprüchen enthalten.

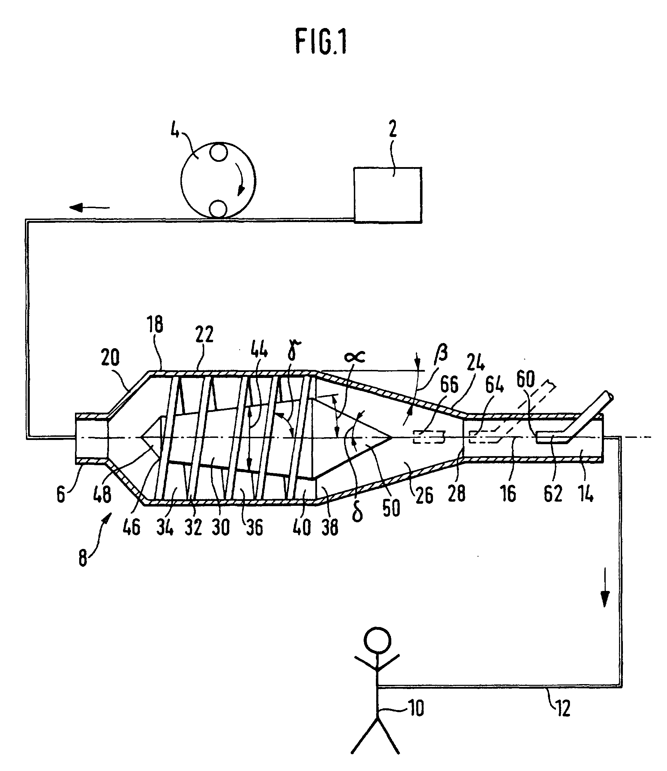

Die Erfindung wird im folgenden mit Bezug auf die Zeichnung anhand einer bevorzugten Ausführungsform als Beispiel beschrieben. In der Zeichnung zeigt
- Fig. 1: schematisch und unmaßstäblich, teilweise im Axialschnitt, eine Vorrichtung nach der Erfindung zum Ausscheiden von Gas aus gashaltigem Blut.

Fig. 1 zeigt eine Blutquelle 2, welche eine in der Medizin bekannte Vorrichtung sein kann, beispielsweise ein Blut-Oxygenator, eine Herz-Lungen-Maschine, ein Blutfilter, ein Reservoir, ein Cardioplegie-System, ein Plasmaphorese-System, ein Dialyse-System oder ein anderes Bluttransfusionssystem. Die Blutquelle ist über eine Druckpumpe 4 an einen Eingang 6 einer Zyklon-Wirbelstromvorrichtung 8 angeschlossen und fördert durch diese hindurch Blut zu einem Patienten 10 oder einem Gerät. Dieses Gerät oder der Patient 10 ist über eine Schlauchleitung 12 an einen Zyklon-Auslaßkanal 14 der Zyklon-Wirbelstromvorrichtung 8 angeschlossen.

Die Zyklon-Wirbelstromvorrichtung 8 enthält axial nacheinander und koaxial zu einer geraden Mittelachse 16 angeordnet ein im Querschnitt kreisrundes Gehäuse 18 mit dem Eingang 6 am einen axialen Ende und dem Auslaßkanal 14 am anderen axialen Ende. Das Gehäuse 18 hat axial nacheinander am Eingang 6 einen in Strömungsrichtung trichterartig weiter werdenden Eingangsabschnitt 20, dann einen zylindrischen Kanalabschnitt 22 und anschließend einen in Strömungsrichtung trichterartig enger werdenden Zyklon-Wirbelkammerabschnitt 24, welcher die Umfangswand einer in gleicher Weise trichterartig enger werdenden Zyklon-Wirbelkammer 26 bildet. In der Zyklon-Wirbelkammer 26 rotiert das gashaltige Blutgemisch mit gleichbleibender Drehrichtung vom axialen Kammeranfang bis zum axialen Kammerende. Dabei wird das Blutgemisch durch Zentrifugalkräfte in eine radial äußere Blutphase (Blutanteil) und eine radial innere Gasphase (Gasanteil) getrennt. Das stromabwärtige Ende 28 der Zyklon-Wirbelkammer 26 ist mit dem stromaufwärtigen Anfang des Auslaßkanals 14 verbunden und bildet einen Zyklonauslaß für die Blutphase.

Im Kanalabschnitt 22 des Gehäuses 18 ist koaxial zur Mittelachse 16 ein Einsatzkörper 30 eingesetzt, welcher mindestens eine gewindeartig verlaufende Rippe 32 und zwischen ihr mindestens eine gewindeartig gebildete Nut 34 aufweist, welche zusammen mit dem Gehäuse 18 einen gewindeförmigen Bluteinlaßkanal 36 begrenzen. Der Bluteinlaßkanal 36 erstreckt sich von einer Stelle stromabwärts des Eingangs 6 bis zum stromaufwärtigen Anfang 38 der Zyklon-Wirbelkammer 26 und bildet dort einen im wesentlichen tangentialen Zykloneinlaß 40, aus welchem das gashaltige Blut im wesentlichen tangential in die Zyklon-Wirbelkammer 26 strömt und in Form eines Zyklon-Wirbelstromes bis zu dem Kammerende 28 strömt, durch welches es weiterhin rotierend in den Auslaßkanal 14 gelangt. Die Zyklon-Wirbelkammer 26 kann über ihre gesamte Länge entsprechend Fig. 1 trichterförmig verengt ausgebildet sein oder zumindest an ihrem stromaufwärtigen Anfangsabschnitt eine kreiszylindrische Form haben. Die trichterartig enger werdende Form der Zyklon-Wirbelkammer 26 hat den Zweck, die Zyklon-Zentrifugalenergie über die gesamte axiale Länge der Zyklon-Wirbelkammer 26 aufrechtzuerhalten.

Der Durchmesser 44 des Einsatzkörpers 30 am Grund der Nuten 34 ist am stromaufwärtigen Nutenanfang 46 am kleinsten und wird in Strömungsrichtung bis zum Zykloneinlaß 40 hin stromabwärts zunehmend größer. Der Kanalabschnitt 22 des Gehäuses 18, welcher die Nuten 34 am Außenumfang begrenzt, kann statt einer kreiszylindrischen Form eine andere Form derart haben, daß der durch die Rippen 32, die Nuten 34 und den Kanalabschnitt 22 begrenzte gewindeartige Bluteinlaßkanal 36 mindestens auf einem Teil seiner Länge, vorzugsweise aber auf seiner gesamten Länge, einen in Strömungsrichtung trichterartig kontinuierlich kleiner werdenden Strömungsquerschnitt hat, so daß das gashaltige Blut in ihm stromabwärts beschleunigt wird und mit möglichst großer Geschwindigkeit in die Zyklon-Wirbelkammer 26 strömt.

Die Rippen 32 können am Kanalabschnitt 22 anliegen oder einen kleinen Abstand davon haben. Der Einsatzkörper 30 hat vorzugsweise an seinem stromaufwärtigen Anfang eine gegen die Blutströmung gerichtete Kegelspitze 48 und an seinem stromabwärtigen Ende eine in Strömungsrichtung gerichtete Kegelspitze 50. Anstelle solcher Kegelspitzen 48 und 50 kann der Einsatzkörper 30 auch gerundete oder flache Stirnflächen haben.

Die in der Zeichnung angegebenen Winkel haben vorzugsweise folgende Größenbereiche: Winkel α zwischen der Mittellinie 16 und einer Mantellinie des Einsatzkörpers 30 auf dem Grund der Nuten 34 0° bis 30°; Winkel β zwischen dem Kanalabschnitt 22 des Gehäuses 18 und dem Zyklon-Wirbelkammerabschnitt 24 des Gehäuses 18 0° bis 45°; der Winkel γ zwischen der Mittelline 16 und einer Stirnseite der Rippen 32 quer zur Mittellinie 16 45° bis 80°; und der Winkel δ der stromabwärtigen Kegelspitze 50 zwischen der Mittellinie 16 und der Mantellinie dieser Kegelspitze 50 von 90° bis 15°. Wenn der Winkel α zwischen der Mittellinie 16 und der Längslinie auf dem Grund der Nuten 34 0° oder nur wenige Grade beträgt, dann muß die Längslinie des Kanalabschnitts 22 in Blutströmungsrichtung schräg auf die Mittellinie 16 hin zulaufen, damit die Nuten 34 des Bluteinlaßkanals 36 in Blut-Strömungsrichtung eine keilförmig enger werdende Querschnittsgröße haben. Eine andere Möglichkeit, die Nuten 34 und damit auch den Bluteinlaßkanal 36 in Strömungsrichtung trichterartig enger werdend auszubilden besteht darin, den Abstand ihrer Rippen 32 in Strömungsrichtung kontinuierlich kleiner werdend auszubilden. Dadurch kann die Höhe und/oder die Breite der Nuten 34 verändert werden.

Das am Zykloneinlaß 40 tangential in die Zyklon-Wirbelkammer 26 einströmende gashaltige Blut strömt in Form eines Zyklon-Wirbelstromes durch die Zyklon-Wirbelkammer 26 zu deren Auslaßende 28. Dabei werden Zentrifugalkräfte erzeugt, welche die Blutphase oder den Blutanteil des gashaltigen Blutes in den radial äußeren Zyklon-Wirbelstrombereich drängen. Dabei drängt die Blutphase, welche schwerer ist als das im Blut enthaltene Gas, dieses Gas oder diese Gasphase in den radial inneren Zyklon-Wirbelstrombereich. Die Zyklon-Wirbelströmung wandert in den Auslaßkanal 14. Im radial inneren Zentrum dieser Zyklon-Wirbelströmung ist eine Gasauslaßöffnung 60 koaxial zur Mittellinie 16 entgegengesetzt zur axialen Strömung der Blutphase und der Gasphase angeordnet, so daß die Gasphase nur aus einem kleinen Querschnittsbereich in und um die Mittellinie 16 herum in die Gasauslaßöffnung 60 strömen kann. Die Gasauslaßöffnung 60 kann beispielsweise bis zu 10 cm stromabwärts des stromabwärtigen Endes 28 der Zyklon-Wirbelkammer 26 angeordnet sein, wie dies durch eine koaxial im Auslaßkanal 14 angeordnete Gasleitung 62 dargestellt ist, oder am stromabwärtigen Ende 28 der Zyklon-Wirbelkammer 26, wie dies bei einer Bezugszahl 64 gestrichelt dargestellt ist, oder stromaufwärts dieses Endes 28, wie dies bei einer Bezugszahl 66 schematisch dargestellt ist. In allen Fällen befindet sich die Gasauslaßöffnung 60 koaxial zur Mittellinie 16 und ist entgegen der axialen Strömungsrichtung der Gasphase und der Blutphase gerichtet.

Bei der Ausführungsform von Fig. 1 sind der Eingang 6, die Wirbelkammer 26, der Auslaßkanal 14, die Gasauslaßöffnung 60 und mindestens der Anfangsabschnitt der Gasleitung 62, in welchem die Gasauslaßöffnung 60 gebildet ist, koaxial zur geraden Mittellinie 16 angeordnet.

Gemäß abgewandelten Ausführungformen nach der Erfindung kann die Richtung des Eingangs 6 in einem Bereich liegen, welcher zwischen einer axialen und einer tangentialen Richtung zur Mittellinie 16 liegt, wobei die tangentiale Richtung in die gleiche Umfangsrichtung zeigt wie die Nuten 34, so daß die Blutströmung nicht umgekehrt wird. Ferner kann die Richtung des Blut-Auslaßkanals 14 und/oder die Richtung der Gasauslaßöffnung 60 und ihrer Gasleitung 62, oder mindestens des Anfangsabschnitts von dieser Gasleitung 62, in einem Bereich zwischen der axialen Vorwärtsrichtung gemäß Fig. 1 und der tangentialen Bewegungsrichtung des Zyklon-Wirbelstromes liegen.

## Patentansprüche

1. Vorrichtung zum Ausscheiden von Gas aus gashaltigem Blut, mit folgenden Merkmalen: eine nicht-rotierende Zyklon-Wirbelkammer (26), durch welche gashaltiges Blut in Form eines Zyklon-Wirbelstromes hindurchleitbar ist, um es durch Zentrifugalkräfte in eine Blutphase im radial äußeren Zyklon-Wirbelstrombereich und eine Gasphase im radial inneren Zyklon-Wirbelstrombereich zu trennen; die Zyklon-Wirbelkammer (26) hat einen Zykloneinlaß (40) für das gashaltige Blut und mit axialem Abstand davon einen Zyklonauslaß (28) für die Blutphase; es ist ein Gasauslaß (60) zum Ableiten der Gasphase getrennt von der Blutphase vorgesehen; der Zykloneinlaß (40) und der Zyklonauslaß (28) für die Blutphase sind derart angeordnet, daß der Zyklon-Wirbelstrom ohne Umkehrung seiner Strömungsrichtung vom Zykloneinlaß (40) zum Zyklonauslaß (28) rotiert;
**dadurch gekennzeichnet, daß** der Zykloneinlaß (40) durch mindestens einen gewindeartig kreisförmig verlaufenen Bluteinlaßkanal (36) gebildet ist, welcher mindestens auf einem Teil seiner Länge in Strömungsrichtung trichterartig enger werdend ausgebildet ist, um den Strom des gashaltigen Blutes zu beschleunigen, und welcher einen im wesentlichen tangential in die Zyklon-Wirbelkammer (26) gerichteten Endabschnitt hat; daß der Gasauslaß (60) stromabwärts des Bluteinlaßkanals (36) im radial inneren Zentrum des Zyklon-Wirbelstromweges angeordnet ist und sich in einer Richtung erstreckt, die im Bereich zwischen axialer Vorwärtsrichtung und tangentialer Bewegungsrichtung des Zyklon-Wirbelstromes liegt.

2. Vorrichtung nach Anspruch 1,
**dadurch gekennzeichnet, daß** der Gasauslaß (60) für die Gasphase und der Zyklonauslaß (28) für die Blutphase koaxial ineinander angeordnet sind.

3. Vorrichtung nach Anspruch 2,
**dadurch gekennzeichnet, daß** der Gasauslaß (60) stromabwärts des Zyklonauslasses (28) für die Blutphase in einem auf diesen Zyklonauslaß folgenden Auslaßkanal (14) angeordnet ist.

4. Vorrichtung nach einem der Ansprüche 1 bis 3,
**dadurch gekennzeichnet, daß** der Bluteinlaßkanal (36) in einem Gehäuse (18) zwischen einer Gehäusewand (22) und einem in das Gehäuse (18) eingesetzten Einsatzkörper (30) gebildet ist.

5. Vorrichtung nach Anspruch 4,
**dadurch gekennzeichnet, daß** der Einsatzkörper (30) mindestens eine gewindeartig verlaufende Rippe (32) und eine zwischen ihr gebildete gewindeartig verlaufende Nut (34) aufweist, die zusammen mit der Gehäusewand (22) den Bluteinlaßkanal (36) bilden.

6. Vorrichtung nach Anspruch 5,
**dadurch gekennzeichnet, daß** der Durchmesser des Einsatzkörpers (30) am Grund der Nut (34) von einem kleinsten Durchmesser am stromaufwärtigen Anfang bis zu einem größten Durchmesser am stromabwärtigen Ende kegelartig größer werdend ausgebildet ist.

7. Vorrichtung nach Anspruch 5 oder 6,
**dadurch gekennzeichnet, daß** der Einsatzkörper (30) durch seine Rippe (32) an der Gehäusewand (22) abgestützt ist.

8. Vorrichtung nach einem der Ansprüche 5 bis 7,
**dadurch gekennzeichnet, daß** der Einsatzkörper (30) an seinem stromaufwärtigen Anfang eine entgegen der Blutströmungsrichtung gerichtete zentrale Kegelspitze (48) hat.

9. Vorrichtung nach einem der Ansprüche 5 bis 8,
**dadurch gekennzeichnet, daß** der Einsatzkörper (30) an seinem stromabwärtigen Ende eine in die Zyklon-Wirbelkammer (26) hineinragende Kegelspitze (50) hat.

10. Vorrichtung nach einem der Ansprüche 1 bis 9,
**dadurch gekennzeichnet, daß** die Mittelachse (16) der Zyklon-Wirbelkammer (26) und die Mittelachse der Gewindeform des Bluteinlaßkanals (36) miteinander fluchten.

11. Vorrichtung nach einem der Ansprüche 1 bis 10,
**dadurch gekennzeichnet, daß** die Mittelachse (16) der Zyklon-Wirbelkammer (26) mit der Mittelachse des Zyklonauslasses (28) für die Blutphase fluchtet.

12. Vorrichtung nach einem der Ansprüche 1 bis 11,
**dadurch gekennzeichnet, daß** die Zyklon-Wirbelkammer (26) mindestens auf ihrem stromabwärtigen Endabschnitt in Strömungsrichtung trichterartig enger werdend ausgebildet ist.

13. Vorrichtung nach einem der Ansprüche 1 bis 12,
**dadurch gekennzeichnet, daß** eine das Blut mit Überdruck fördernde Druckpumpe (4) vorgesehen ist.

## Claims

1. Device for separating gas from gaseous blood, having the following features: a non-rotating cyclone vortex chamber (26) through which gaseous blood can be passed in the form of a cyclone vortex flow in order to separate it by means of centrifugal forces into a blood phase in the radially outer cyclone vortex flow region and a gas phase in the radially inner cyclone vortex flow region; the cyclone vortex chamber (26) has a cyclone inlet (40) for the gaseous blood and a cyclone outlet (28) for the blood phase at an axial distance therefrom; a gas outlet (60) is provided for discharging the gas phase separately from the blood phase; the cyclone inlet (40) and the cyclone outlet (28) for the blood phase are arranged in such a manner that the cyclone vortex flow rotates without reversing its flow direction from the cyclone inlet (40) to the cyclone outlet (28), **characterised in that** the cyclone inlet (40) is formed by at least one threaded circular blood-inlet channel (36) which tapers in the flow direction at least over part of its length in order to accelerate the flow of the gaseous blood and has an end portion directed substantially tangentially into the cyclone vortex chamber (26), that the gas outlet (60) is arranged downstream of the blood-inlet channel (36) in the radially inner centre of the cyclone vortex flow path and extends in a direction situated in the region between the axial forward direction and the direction of the tangential movement of the cyclone vortex flow.

2. Device according to claim 1, **characterised in that** the gas outlet (60) for the gas phase and the cyclone outlet (28) for the blood phase are arranged coaxially one inside the other.

3. Device according to claim 2, **characterised in that** the gas outlet (60) is arranged downstream of the cyclone outlet (28) for the blood phase in an outlet channel (14) following this cyclone outlet.

4. Device according to one of claims 1 to 3, **characterised in that** the blood-inlet channel (36) is formed in a housing (18) between a housing wall (22) and an insert body (30) inserted in the housing (18).

5. Device according to claim 4, **characterised in that** the insert body (30) has at least one threaded rib (32) and a threaded groove (34) formed therebetween, forming the blood-inlet channel (36) together with the housing wall (22).

6. Device according to claim 5, **characterised in that** the diameter of the insert body (30) at the bottom of the groove (34) flares out from a smallest diameter at the upstream front end to a largest diameter at the downstream end.

7. Device according to claim 5 or claim 6, **characterised in that** the insert body (30) is supported on the housing wall (22) by its rib (32).

8. Device according to one of claims 5 to 7, **characterised in that** the insert body (30) is provided at its upstream front end with a central cone vertex (48) opposite to the direction of the blood flow.

9. Device according to one of claims 5 to 8, **characterised in that** the insert body (30) is provided at its downstream end with a cone vertex (50) projecting into the cyclone vortex chamber (26).

10. Device according to one of claims 1 to 9, **characterised in that** the centre axis (16) of the cyclone vortex chamber (26) and the centre axis of the threaded shape of the blood-inlet channel (36) are aligned with one another.

11. Device according to one of claims 1 to 10, **characterised in that** the centre axis (16) of the cyclone vortex chamber (26) is aligned with the centre axis of the cyclone outlet (28) for the blood phase.

12. Device according to one of claims 1 to 11, **characterised in that** the cyclone vortex chamber (26) tapers in the flow direction at least at its downstream end portion.

13. Device according to one of claims 1 to 12, **characterised in that** a pump (4) supplying the blood with excess pressure is provided.

## Revendications

1. Appareil pour séparer un gaz d'un sang contenant du gaz, possédant les caractéristiques suivantes : une chambre de turbulence de cyclone non rotative (26), par laquelle du sang contenant du gaz peut être introduit sous la forme d'un écoulement tourbillonnaire de cyclone, afin de le séparer par des forces centrifuges en une phase sanguine dans la zone radiale exteme de l'écoulement tourbillonnaire de cyclone, et en une phase gazeuse dans la zone radiale interne de l'écoulement tourbillonnaire de cyclone ; la chambre de turbulence de cyclone (26) présente une entrée de cyclone (40) pour le sang contenant du gaz et, selon un écart axial par rapport à celle-ci, une sortie de cyclone (28) pour la phase sanguine ; une sortie de gaz (60) permettant d'évacuer la phase gazeuse séparément de la phase sanguine est prévue ; l'entrée de cyclone (40) et la sortie de cyclone (28) pour la phase sanguine sont disposées de façon telle que l'écoulement tourbillonnaire de cyclone toume sans inversion de sa direction d'écoulement de l'entrée de cyclone (40) vers la sortie de cyclone (28) ;
**caractérisé en ce que** l'entrée de cyclone (40) est formée par au moins un canal d'entrée de sang (36) de type filet s'étendant pour produire un cercle, lequel canal est conçu au moins sur une partie de sa longueur en se rétrécissant en forme d'entonnoir dans la direction de l'écoulement, afin d'accélérer l'écoulement du sang contenant du gaz, et lequel canal possède un segment d'extrémité orienté essentiellement tangentiellement vers la chambre tourbillonnaire de cyclone (26) ; **en ce que** la sortie de gaz (60) est disposée en aval du canal d'entrée de sang (36) au centre radial interne de la voie d'écoulement tourbillonnaire du cyclone et s'étend dans une direction qui est située dans une zone entre la direction axiale avant et la direction de déplacement tangentielle de l'écoulement tourbillonnaire du cyclone.

2. Appareil selon la revendication 1, **caractérisé en ce que** la sortie de gaz (60) pour la phase gazeuse et la sortie de cyclone (28) pour la phase sanguine sont disposées l'une dans l'autre de façon coaxiale.

3. Appareil selon la revendication 2, **caractérisé en ce que** la sortie de gaz (60) est disposée en aval de la sortie de cyclone (28) pour la phase sanguine, dans un canal de sortie (14) suivant cette sortie de cyclone.

4. Appareil selon l'une des revendications 1 à 3, **caractérisé en ce que** le canal d'entrée de sang (36) est formé dans un logement (18) entre une paroi de logement (22) et un corps de charge (30) implanté dans le logement (18).

5. Appareil selon la revendication 4, **caractérisé en ce que** le corps de charge (30) présente au moins une nervure (32) s'étendant en forme de filetage et une rainure (34) s'étendant en forme de filetage entre les filets de nervure, pour former avec la paroi de logement (22) le canal d'entrée de sang (36).

6. Appareil selon la revendication 5, **caractérisé en ce que** le diamètre du corps de charge (30) à la base de la rainure (34) est conçu en cône s'élargissant depuis un plus petit diamètre au départ en amont jusqu'à un plus grand diamètre à l'extrémité aval.

7. Appareil selon la revendication 5 ou 6, **caractérisé en ce que** le corps de charge (30) est supporté contre la paroi de logement (22) par sa nervure (32).

8. Appareil selon l'une des revendications 5 à 7, **caractérisé en ce que** le corps de charge (30) possède au niveau de son départ en amont une pointe (48) centrale orientée à l'opposé de la direction d'écoulement du sang.

9. Appareil selon l'une des revendications 5 à 8, **caractérisé en ce que** le corps de charge (30) possède au niveau de son extrémité en aval une pointe (50) faisant saillie dans la chambre de turbulence de cyclone (26).

10. Appareil selon l'une des revendications 1 à 9, **caractérisé en ce que** l'axe médian (16) de la chambre de turbulence de cyclone (26) et l'axe médian de la forme en filetage du canal d'entrée de sang (36) sont alignés.

11. Appareil selon l'une des revendications 1 à 10, **caractérisé en ce que** l'axe médian (16) de la chambre de turbulence de cyclone (26) est aligné sur l'axe médian de la sortie de cyclone (28) pour la phase sanguine.

12. Appareil selon l'une des revendications 1 à 11, **caractérisé en ce que** la chambre de turbulence de cyclone (26) est conçue au moins sur son segment d'extrémité en aval en se rétrécissant en entonnoir dans la direction de l'écoulement.

13. Appareil selon l'une des revendications 1 à 12, **caractérisé en ce qu'**une pompe refoulante (4) transportant le sang par surpression est prévue.
